# EUROPEAN PATENT APPLICATION

(11) **EP 2 315 017 A1**
(43) Date of publication of application: **27.04.2011**
(21) Application number: 09794406.0
(22) Date of filing: 06.07.2009
(51) Int. Cl.: G01N 27/62, G01N 33/15, G01N 33/50, C07K 7/06, C07K 7/08, C07K 14/47, C07K 14/75, C07K 14/775, C07K 14/81

(54) **METHOD FOR DETECTION OF FIBROMYALGIA**

(30) Priority: 07.07.2008 JP 2008176704
(71) Applicant: Nippon Zoki Pharmaceutical Co., Ltd., Osaka-shi Osaka 541-0046 (JP)
(72) Inventor: NISHIOKA, Kusuki, Tokyo 150-0012 (JP); KATO, Tomohiro, Kawasaki-shi Kanagawa 216-0005 (JP); FUJISAWA, Hiroki, Kato-shi Hyogo 673-1461 (JP)
(74) Representative: Siegert, Georg
(86) International application number: PCT/JP2009/062305
(87) International publication number: WO 2010/004962

(57) **Abstract**

An object of the present invention to provide a method for diagnosing or testing for fibromyalgia with a specific peptide in the blood as an indicator, as well as a method for effectively evaluating or assessing a fibromyalgia drug with the peptide as an indicator, The present invention is useful as a methods for diagnosing or testing for fibromyalgia or for evaluating or assessing a fibromyalgia drug, by subjecting a patent's serum to peptide analysis using as an indicator (biomarker) a peptide that demonstrates a specific expression amount in the blood of a fibromyalgia patient.

SEQUENCE LISTING

<110> Nippon Zoki Pharmaceutical Co., Ltd.

<120> Examination method of fibromyalgia

<130> FP-581

<150> JP 2008-176704
<151> 2008-07-07

<160> 15

<210> 1
<211> 17
<212> PRT
<213> Homo sapiens

<220>
<223> Inventor: NISHIOKA, Kusuki; KATO, Tomohiro; FUJISAWA, Hiroki

<400> 1
Gly Leu Pro Gly Pro Pro Asp Val Pro Asp His Ala Ala Tyr His Pro
1               5                   10                  15
Phe

<210> 2
<211> 17
<212> PRT
<213> Homo sapiens

<400> 2
Asn Leu Gly His Gly His Lys His Glu Arg Asp Gln Gly His Gly His
1               5                   10                  15

Gln

<210> 3
<211> 19
<212> PRT
<213> Homo sapiens

<400> 3
Gln Leu Gly Leu Pro Gly Pro Pro Asp Val Pro Asp His Ala Ala Tyr
1               5                   10                  15

His Pro Phe

<210> 4
<211> 18
<212> PRT
<213> Homo sapiens

<400> 4
His Asn Leu Gly His Gly His Lys His Glu Arg Asp Gln Gly His Gly
1               5                   10                  15

His Gln

<210> 5
<211> 20
<212> PRT
<213> Homo sapiens

<400> 5
Gly His Gly Leu Gly His Gly His Glu Gln Gln His Gly Leu Gly His
1               5                   10                  15

Gly His Lys Phe
            20

<210> 6
<211> 19
<212> PRT
<213> Homo sapiens

<400> 6
Lys His Asn Leu Gly His Gly His Lys His Glu Arg Asp Gln Gly His
1               5                   10                  15

Gly His Gln

<210> 7
<211> 21
<212> PRT
<213> Homo sapiens

<400> 7
Thr Val Gly Ser Leu Ala Gly Gln Pro Leu Gln Glu Arg Ala Gln Ala
1               5                   10                  15

Trp Gly Glu Arg Leu
            20

<210> 8
<211> 10
<212> PRT
<213> Homo sapiens

<400> 8
Asp Phe Leu Ala Glu Gly Gly Gly Val Arg
1               5                   10

<210> 9
<211> 12
<212> PRT
<213> Homo sapiens

<400> 9
Glu Gly Asp Phe Leu Ala Glu Gly Gly Gly Val Arg
1               5                   10

<210> 10
<211> 14
<212> PRT
<213> Homo sapiens

<400> 10
Ser Gly Glu Gly Asp Phe Leu Ala Glu Gly Gly GLy Val Arg
1               5                   10

<210> 11
<211> 15
<212> PRT
<213> Homo sapiens

<400> 11
Asp Ser Gly Glu Gly Asp Phe Leu Ala Glu Gly Gly Gly Val Arg
1               5                   10                  15

<210> 12
<211> 16
<212> PRT
<213> Homo sapiens

<400> 12
Ala Asp Ser Gly Glu Gly Asp Phe Leu Ala Glu Gly Gly Gly Val Arg
1               5                   10                  15

<210> 13
<211> 17
<212> PRT
<213> Homo sapiens

<400> 13
Thr Ala Asp Ser Gly Glu Gly Asp Phe Leu Ala Glu Gly Gly Gly Val
1               5                   10                  15

Arg

<210> 14
<211> 8
<212> PRT
<213> Homo sapiens

<400> 14
His Trp Glu Ser Ala Ser Leu Leu
1               5

<210> 15
<211> 23
<212> PRT
<213> Homo sapiens

<400> 15
Ala Leu Gly Ile Ser Pro Phe His Glu His Ala Glu Val Val Phe Thr
1               5                   10                  15

Ala Asn Asp Ser Gly Pro Arg
            20

## Description

### TECHNICAL FIELD

The present invention relates to a method for diagnosing or testing for fibromyalgia using a specific peptide in blood as an indicator (biomarker), as well as a method for evaluating or assessing a fibromyalgia drug using the peptide as an indicator, and the like,

### BACKGROUND ART

In recent years, methods such as transcriptome analysis and proteome analysis have been developed in life science research to analyze comprehensively gene products such as messenger RNAs and proteins. In particular, such results as novel disease markers and therapeutic targets being discovered have been obtained with proteome analysis, Meanwhile, among the peptides are those that participate in vivo in various reactions as hormones, circulation regulators, neuro-transmitters/regulators, and the like, assuming signal transduction/regulation in an organism, and as of recently, it is found that disease-specific small peptides exist. Therefore, similarly to proteome analysis and the like, peptidome analysis is becoming to be promising as a useful method for developing disease markers, elucidating pathologies and determining novel therapeutic targets.

Fibromyalgia (FM, also called as Fibromyalgia Syndrome, FMS; hereinafter referred to as "fibromyalgia" or "FM") is a disease in which the main symptom is chronic, strongly systemic pain, or, even if partial, widespread chronic pain, the pain being observed not only in muscular tissues but also in the skin. In fibromyalgia, such systemic chronic pain is often not alone and is also accompanied by a feeling of fatigue, malaise, depression, a feeling of anxiety, a feeling of morning stiffness, muscle stiffness, sleep disturbance or the like. In addition, symptoms such as headache, facial pain, cognitive impairment (lapse of memory, concentration deficit), gastrointestinal complaints (visceral pain, digestive system disturbance, flatulence), frequent urination, diarrhea constipation or dysmenorrhea may also occur concomitantly,

It has been reported that the prevalence of fibromyalgia was 2% in the general population of the USA (3.4% of females, 0.5% of males) and 2,7% of the general population of Canada (4.9% of females, 1.6% of males). It has been learned that, also in Japan, the prevalence rate is 1.7% in population ratio, a frequency approximately comparable to the USA. This disease occurs commonly in females aged 25 to 50, and approximately 80% of the patients are females. With fibromyalgia, although subjective symptoms are diverse, there are not many objective findings other than the characteristic whole body tenderness, and almost no anomalies can be observed even if, in addition to imaging tests such as MRI and CT, pathological examination of the site of muscle pain, various immunological, virological and endocrinological examinations are carried out. For instance, unlike rheumatoid arthritis no edema is observed, and although blood indicators that indicate the extent of the inflammation, that is to say, sedimentation rate and CRP, are in normal ranges, patients complain of pain over a wide-range in the limbs and the trunk.

Regarding the cause or the mechanism of the onset of fibromyalgia, psychological factors such as stress, viral infection, inheritance, anomaly in immunity, anomaly in neurotransmitter and the like are currently inferred; however, it is still not understood. Fibromyalgia is a disease that is extremely different from a number of general painful diseases exerted by nociceptive stimuli in which a damage or a possibility of damage of biological tissue exists, and no related pathological finding is observed at the site of pain.

Not much effect has been observed in treating fibromyalgia for most of the antiphlogistic analgesics frequently used in the treatment of pain in general, such as nonsteroidal anti-inflammatory drug (NSAIDs). In addition, although various drugs such as muscle relaxants, opioid analgesics and anti-anxiety drugs are under trial use, there are large individual differences in the effectiveness thereof and no prominent effect has been observed. Consequently, treatment of fibromyalgia currently merely integrates the prescription of an antidepressant or of this and NSAIDs, the administration of a local anesthetic or of a steroidal agent to the trigger site, massages, exercise therapy, sleep therapy and the like. However, for all the therapeutic agents and methods, the cause of fibromyalgia may not be specified, there are large individual differences in the therapeutic effects, such that they are not established as therapeutic methods.

As diagnostic methods for fibromyalgia, at the current stage, common ones are based on the classification criteria proposed in 1990 year by the American Colloge, of Rheumatology. With these criteria, a case where pain is identified in all the sites from among five locations, that is, the upper body and the lower body, the right and the left sides of the body, and the spine region or the breast bone region, the umbilical region serving as the base point, and these persist for at least three months or more, or, a case where a gentle load of 4 kg is applied on tender points at 18 locations defined throughout the body, and pain is felt at 11 or more locations, is deemed fibromyalgia. Other than such diagnosis by tender point stimulation, there are no diagnostic tests for fibromyalgia, since there is no anomaly even when examining blood, x-rays, CRP (inflammatory response), electromyogram, muscle enzymes CT and MRI are examined, such that the situation is that many patients continue to visit several medical institutes over many years until being diagnosed.

As stated above, currently, owing to the fact that the cause and the mechanism of the onset of fibromyalgia is not clear, there is no clinical test method allowing fibromyalgia to be diagnosed accurately with a specific indicator, and in addition, there is no method for effectively evaluating or assessing whether or not a drug is effective for fibromyalgia either. Therefore, such diagnosis or test method and evaluation or assessment method are sought in the field of medicine.

No related art that is related to the present invention exists,

### DISCLOSURE OF THE INVENTION

### Problem to be Solved by the Invention

An object of the present invention to provide a method for diagnosing or testing for fibromyalgia with a specific peptide in the blood as an indicator, as well as a method for effectively evaluating or assessing a fibromyalgia drug with the peptide as an indicator.

### Means for Solving the Problem

The inventors carried out peptidome analysis on blood from fibromyalgia patient and healthy subject, compared the amounts of peptides expressed in both serum samples, and discovered peptides that demonstrate expression amounts that are specific to the disease. The present invention is a method for diagnosing or testing for fibromyalgia using such peptides as indicators (biomarkers).

### Effects of the Invention

The present invention provides a method for diagnosing or testing for fibromyalgia using as an indicator (biomarker) a peptide that demonstrates a specific expression amount in the blood of a fibromyalgia patient. In addition, it provides a method for evaluating or assessing the effect of a test drug on fibromyalgia with the expression amounts in blood of these peptides against fibromyalgia as indicators.

### BEST MODES FOR CARRYING OUT THE MENTION

The present invention relates to a method for diagnosing or testing for fibromyalgia that analyzes specific peptides present in blood (serum) collected from human as indicators (biomarkers), and more concretely, it relates to a method for diagnosing or testing for fibromyalgia, or the like, that carries out peptidome analysis in a sample using a matrix-assisted laser desorption ionization/time of flight mass spectrometer (MALDI-TOF/MS).

The method for diagnosing or testing for fibromyalgia of the present invention can be carried out by peptide analysis methods using frequently-used mass spectrometry or the like. As necessary, the measurement sample may be subjected to a suitable pretreatment, for instance, pretreatment such as dominating high molecular weight substances from the collected serum by ultrafiltration or the like can be carried out. After the pretreatment, absorption on immobilized reversed phase resin tip or the like, elution with a solvent and fraction recovery, the obtained peptide fraction is used as the measurement sample. In the peptide analysis, a method using frequently-used mass spectrometry or the like such as MALDI-TOF/MS can be employed.

In the present invention, diagnosis or test for fibromyalgia or, evaluation or assessment of a fibromyalgia drug can be carried out with any peptide defined in (1) to (10) below as an indicator (biomarker).

(1) A peptide found to have a mass-to-charge ratio (m/z) of 650.0, 656.0, 657.0, 666.0, 672.0, 679.4, 685.4, 737.5, 795.6, 810.6, 825.5, 853.7, 854.1, 861.2, 861.5, 868.4, 890.0, 911.8, 912.1, 921.4, 942.6, 949.3, 964.6, 969.8, 970.5, 1027.9, 1028.7, 1042,5, 1055.9, 1085.9, 1086.7, 1144.8, 1741.1, 1789,60, 1898,8, 1944.7, 2012,2, 2013,7, 2030.8, 2034.1, 2046.6, 2053,4, 2056,1, 201.2, 2073.9, 2083,6, 2118.0, (2130,2), 2187.2, 2212,0, 2253.9, 2268,5, 270,7, 359.5, 2534.1, 2570.5, 2600,8, 2604.1, 2625.4, 2726.2, 2819.4, 3325.9, 3364.2 or 3380.1, as a result of measuring peptides in the blood by mass Spectrometry. These peptides are present in larger amounts in the blood (serum) of an FM patient then a healthy subject.

(2) A peptide found to have a mass-to-charge ratio (m/z) of 615.6,637.4, 1020.6, 1021.3, 1061.6, 1077.8, 1206.8, 1208,0,1262,0, 1350.7, 1350.8, 1419.6, 1420.4, 1465.9, 1467.6, 1521.0, 1547.6, 1562.9, 1563.0, 1564.5, 1618.9, 1972.2, 2452.7, 2452.8, 2454.6, 2646.9, 2770.5, 2934.0, 2981.7, 3193.1 or 3263.9, as a result of measuring peptides in the blood by mass spectrometry. These peptides are present in smaller amounts in the blood (serum) of an FM patient than a healthy subject.

Note that, in the present invention, ±2 m/z is permissible in the values of mass-to-charge ratio in (1) and (2) above, and if within these permitted limits the peptides are recognized as being identical.

(3) A peptide in blood derived from high molecular weight kininogen, fibrinogen, inter-alpha-trypsin inhibitor H4, apolipoprotein E, complement C3f or transthyretin A chain.

(4) A peptide in blood corresponding to an amino acid sequence from position 438 to 456, position 439 to 456, position 440 to 456 or position 458 to 477 of high molecular weight kininogen.

(5) A peptide in blood corresponding to an amino acid sequence from position 19 to 35, position 20 to 35, position 21 to 35, position 22 to 35, position 24 to 35, position 26 to 35 or position 28 to 35 of fibrinogen alpha chain.

(6) A peptide in blood corresponding to an amino acid sequence from position 626 to 642 or position 669 to 687 of inter-alpha-trypsin inhibitor H4.

(7) A peptide in blood corresponding to an amino acid sequence from position 212 to 232 of apolipoprotein E.

(8) A peptide in blood corresponding to an amino acid sequence from position 9 to 16 of complement C3f.

(9) A peptide in blood corresponding to an amino acid sequence from position 101 to 123 of transthyretin A chain.

(10) A peptide in blood the amino acid sequence of which is any one of SEQ ID NO. 1 to 15.

### Examples

A peptidome analysis system Clinprot System (Bruker Daltonics) with a matrix-assisted laser desorption ionization/time of flight mass spectrometer (MALDI-TOF/MS) was used to carry out peptidome analysis for sera collected from 19 patients diagnosed as fibromyalgia (FM), as outlined below. Note that sera from 20 healthy subjects were used normal controls, and in addition, all reagents used were of mass spectrometry grade as recommended by Bruker Daltonics.

### (1) Simple preparation

### A) Samples diluted with distilled water (Sample A)

The sera from FM patients and the sera from healthy subjects were respectively diluted with distilled water, high molecular weight substances (5,000 Da or greater) were removed by ultrafiltration using a centrifugation filtration filter, and then the residual sera were adsorbed to the immobilized reverse phase resin tip Ziptip C18 and eluted with 50% acetonitrile/0.1%TFA to recover the peptide fractions, which were used to measure the mass spectra. Note that for the purpose of peptide identification, after adsorption to Ziptip C18, peptide fractions eluted stepwise with 10, 20, 30, 40 and 50% acetonitrile/0.1%TFA were used.

### B) Samples diluted with 10% acetonitrile (Sample B)

With the purpose of increasing recovery of peptides bound to carrier proteins, the sera from FM patients and the sera from healthy subjects were respectively diluted with 10% acetonitrile, high molecular weight substances (5,000 Da or greater) were removed by ultrafiltration using a centrifugation filtration filter, and then the residual sera were adsorbed to the immobilized reverse phase resin tip Ziptip C18 and eluted with 30% or 60% acetonitrile/0.1%TFA to recover the peptide fractions, which were used to measure the mass spectra.

### (2) Mass spectra measurement by MALDI-TOF/MS

The peptides fractions (samples A and B) obtained in (1) above were respectively fixed with a matrix solution (a-Cyaiio-4-Hydoroxycinnamic Acid), coated over a target plate, and MALDI-TOF/MS (Ultraflex TOF/TOF, Bruker Daltonics) was used to measure the mass spectra (measurement rangers: m/z of 800 to 3,500 for Sample A; m/z of 600 to 3,500 for Sample B).

### (3) Detection and identification of expression variation peaks

The mass spectra measured in (2) above were analyzed using ClinPro Tool 2 (Bruker Daltonics), the sera from FM patients and the sera from healthy subjects were compared, and the peaks for which a variation in the amount of expression was observed were recognized as signals. For these peptides signals, the amino acid sequences were analyzed with Post Source Decay (PSD) MS/Ms, and identification was carried out using the protein/peptide identification software Mascot (Matrix Science).

The results of the above tests were as following.

### (A) Sample A

As a result of comparing/analyzing the FM patient serum samples and the healthy subject serum samples, 72 peptide signals were detected. Among these, there were 31 peptide signals for which the expression amounts were significantly high in the FM patients compared to the healthy subjects, among which four were identified, two being sequences derived from high molecular weight (HMW) kininogen, one derived from inter-alpha-trypsin inhibitor H4 (ITIH4) and one derived from apolipoprotein E (ApoE). Meanwhile, there were 17 peptide signals for which the expression amounts were significantly low in the FM patients compared to the healthy subjects, among which six were identified, all being sequences derived from fibrinogen.

In addition, for the three signals which were not recognized as signals with Clinpro tool 2 but for which a tendency to increase in FM patients was observed (indicated in the following by mass-to-charge ratios with parentheses), two were sequences derived from HMW kininogen and one was derived from ITIH4, The results of the above tests were as follows:

[Peptide signals for which the expression amounts were significantly high in the FM patients.]
810.6, 825.5, 854,1, 861,5, 868,4, 890.0, 912,1, 921.4, 949.3, 970.5, 1028.7, 1042,5, 1055.9, 1086.7, 1144.8, (1789.60), 1898.8, (1944.7), 2013.7, 2030.8, 2046.6, 2053.4, 2073.9, 2083.6, 2118.0, (2130.2), 2187.2, 2212.0, 2270,7, 2534.1, 2570.5, 2604.1, 2625.4, 2819.4

[Peptide signals for which the expression amounts were significantly low in the FM patients.]
1021,3, 1061.6, 1077,8, 1208.0, 1262,0, 1350.8, 1420.4, 1467.6, 1521.0, 1547.6, 1564.5, 1618.9, 2454.6, 2770.5, 2934.0, 3193.1, 3263.9
For the identified peptide signals, an example of the results is shown in Table 1.

**[Table 1]**

| No. | Mass (m/z) | Peptide expression amount | | P value | Origin of the identified sequence |
|---|---|---|---|---|---|
| | | FM patient | Healthy subject | | |
| [Peptide signals for which the expression amounts were significantly high or the expression amounts had a tendency to be high in the FM patients] | | | | | |
| 1 | (1789.60) | (53.09) | (4.92) | - | (ITIH4) |
| 2 | (1944,7) | (49.62) | (13.21) | - | (HMW kininogen) |
| 3 | 2030.8 | 162.82±132,53 | 66.77±102.76 | 0.00124 | ITIH4 |
| 4 | 2083.6 | 11.83±4.63 | 5.69±3.17 | 812E-08 | HMW kininogen |
| 5 | (2130.2) | (43.00) | (25.38) | - | (HMW kininogen) |
| 6 | 2212.0 | 21.01±15.46 | 8.29±9.08 | 0,000125 | HMW kininogen |
| 7 | 2270.7 | 101.92±78.82 | 21.5±16.15 | 1.38E-06 | ApoE |

| [Peptide signals for which the expression amounts were significantly high in the healthy subjects] | | | | | |
|---|---|---|---|---|---|
| 8 | 1021.3 | 3.57±2.59 | 13.06±12.48 | 0.000144 | fibrinopeptide A |
| 9 | 1208.0 | 3.42+2.35 | 42,37±35.98 | 1.21E-06 | |
| 10 | 1350,8 | 35.46±24.4 | 64.32±39.57 | 0.000651 | |
| 11 | 1467.6 | 18.07±11.07 | 144.11±117.86 | 1.21E-06 | |
| 12 | 1521.0 | 10.07±5.71 | 38.82±28.32 | 1.84E-06 | |
| 13 | 1618.9 | 5.63±2.33 | 33.97±27.92 | 1.84E-06 | |

### (B) Sample B

As a result of comparing/analyzing the FM patient serum samples and the healthy subject serum samples, 39 peptide signals were detected in the case of elution with 30% acetonitrile, among these, the expression amounts in the FM patients were significantly high for 11 peptide signals and significantly low for 7 peptide signals. In addition, in the case of elution with 60% acetonitrile, 55 peptide signals were detected, among these, the expression amounts in the FM patients were significantly high for 20 peptide signals and significantly low for 9 peptide signals.

Among these peptide signals, regarding the peptide signals for which the expression amounts in the FM patients were significantly high, one was identified, which was a sequence derived from complement C3f. In addition, regarding the peptide signals for which the expression amounts in the FM patients were significantly low, four were identified, among which three were sequences derived from fibrinopeptide A, which were peptide signals also detected in Sample A, and the retaining one was a sequence derived from transthyretin chain A. The results of the above tests were as follows:

[Peptide signals for which the expression amounts were significantly high in the FM patients.]
650.0, 656.0, 657.0, 666.0, 672.0, 679.4,685.4, 737.5, 795.6, 853.7, 861.2, 911.8, 942.6, 964.6, 969.8, 1027.9, 1085.9, 1741.1, 2012.2, 2071.2, 2034.1, 2056.1, 2253.9, 2268.5, 2359.5, 2600.8, 2726.2, 3325.9, 3364.2, 3380,1

[peptide signals for which the expression amounts were significantly low in the FM patients.]
615.6, 637.4, 1020.6, 1206.8, 1350.7, 1419.6, 1463.9, 1562.9, 1563.0, 1972.2, 2452.7, 2452.8, 2646.9, 2981.7
Regarding the identified peptide signals, an example of these results is shown in Table 2 for the elution with 30% acetonitrile, and in Table 3 for the elution with 60% acetonitrile.

**[Table 2]**

| No. | Mass (m/z) | Peptide expression amount | | P value | Origin of the identified sequence |
|---|---|---|---|---|---|
| | | FM patient | Healthy subject | | |
| [Peptide signals for which the expression amounts were significantly high in the FM patients] | | | | | |
| 14 | 942.6 | 20.79±8.64 | 6.28±4.05 | 1.38E-05 | complement C3f |

| [Peptide signals for which the expression amounts were significantly high in the healthy subjects] | | | | | |
|---|---|---|---|---|---|
| 9 | 1206.8 | 4.75±3.25 | 27.31±22.64 | 0.00187 | fibrinopeptide A |
| 10 | 1350.7 | 8.13±4.25 | 18.47±13.97 | 0.016 | |
| 11 | 1465.9 | 43.03±29.77 | 159.86±111.31 | 0.00131 | |

**[Table 3]**

| No. | Mass (m/z) | Peptide expression amount | | P value | Origin of the identified sequence |
|---|---|---|---|---|---|
| | | FM patient | Healthy subject | | |
| [Peptide signals for which the expression amounts were significantly high in the FM patients] | | | | | |
| 14 | 942.6 | 7.26±3.64 | 2.38±1.83 | 0.00163 | complement C3f |

| [Peptide signals for which the expression amounts were significantly high in the healthy subjects] | | | | | |
|---|---|---|---|---|---|
| 9 | 1206.8 | 1.14±0.49 | 6.07±7.37 | 0.0233 | fibrinopeptide A |
| 11 | 1465.9 | 9.22±4.35 | 35.87±34.3 | 0.0133 | |
| 15 | 2452.7 | 74.33±115.94 | 431.45±500.06 | 0.0198 | TTR chain A |

The amino acid sequence (one letter representation) and SEQ ID NO. of each of the peptide signals shown in above Table 1 to 3 are indicated in Table 4.

**[Table 4]**

| No. | Identified amino acid sequence | |
|---|---|---|
| | One letter notation | Number in the sequence listing |
| 1 | GLPGPPDVPDHAAYHPF | SEQ ID NO.1 |
| 2 | NLGHGHKHBRDQGHGHQ | SEQ ID NO. 2 |
| 3 | QLGLPGPPDVPDHAAYHPF | SEQ ID NO. 3 |
| 4 | HNLGHGHKHERDQGHOHQ | SEQ ID NO. 4 |
| 5 | GHGLGHCHEQQHCLGHCHKF | SEQ ID NO. 5 |
| 6 | KHNLGHGHKHERDQGHGHQ | SEQ ID NO. 6 |
| 7 | TVGSLAGQPLQERAQAWGERL | SEQ 15 NO. 7 |
| 8 | DFLAEGGGVR | SEQ ID NO.8 |
| 9 | EGDFLAEGGGVR | SEQ ID NO. 9 |
| 10 | SGEGDFLAEGGGVR | SEQ ID NO. 10 |
| 11 | DSGEGDFLAEGGGVR | SEQ ID NO. 11 |
| 12 | ADSGEGDFLAEGGGVR | SEQ ID NO. 12 |
| 3 | TADSGEGDFLAEGGGVR | SEQ ID NO. 13 |
| 14 | HWESASLL | SEQ ID NO. 14 |
| 15 | ALGISPFHEHAEVVFTANDSGPR | SEQ ID NO. 15 |

As described above, as a result of comparing/analyzing the FM patient serum samples and the healthy subject serum samples, peptides derived from HMW kininogen, derived from ITIH4, derived from ApoE and derived from complement C3f were identified for the peptides which expression amounts in the FM patients were significantly high, and in addition, peptides derived from fibrinopeptide A and derived from transthyretin chain A were identified for the peptides which expression amounts in the FM patients were significantly low.
The physiological functions and the like of these identified proteins and peptides will be described below.

### (1) Kininogen

Kininogen is a plasma protein composed of 644 amino acids in humans, found to have various physiological functions such as calcium binding, protease inhibition, cell adhesion suppression factor and antithrombotic factor. In addition, it is learned that, from domain 4 of kininogen, bradykinin is generated by kallikrein, inducing pain generation, hemangiectasis, vascular permeability increase, acute inflammation symptoms and the like, The kininogen-derived peptides identified herein are all sequences contained in domain 5, which plays an important role in binding to negatively charged surfaces, have numerous histidines, are extremely rich in charge and exhibit hydrophilicity.

### (2) ITIH4

ITIH4 is a protein composed of 930 amino acids and belonging to the Inter alpha inhibitor family, found to increase in the serum after a trauma or the like, but differs from other molecules from this family on the point that it does not have a bikunin chain, which has protease inhibition activity.

### (3) Apolipoproteins

Apolipoproteins are a group of proteins that bind to lipoproteins and work for activation of or as coenzymes for a group of enzymes involved in the recognition of lipoproteins or in lipid Metabolism. They are broadly classified by structure and function into five species from A to E, and further divided into subclasses. While ApoE is a protein that serves as a marker when lipoproteins axe recognized by cell, relationship to dementia, has been studied in recent years.

### (4) Fibrinopeptide

Herein, for the peptides that could be identified with the expression amount as being low in FM patient serum, most of the sequences were derived from fibrinopeptide A. Fibrinopeptide A is a peptide present in the serum in relatively large amounts, which is cut/produced from the glycoprotein fibrinogen alpha present in large amounts in the plasma by thrombin, and used as an indicator of coagulation promotion start period.

### (5) Complement C3f

This is a peptide fragment excised from C3b which is complement system protein existing in blood.

### (6) TTR chain A

TTR chain A, another protein recognized to have a low value in the FM patients, is present in blood and cerebrospinal fluid and is thought to be a carrier protein for thyroid hormone, retinol and the like. It has been reported that, in TTR-related familial amyloidosis, a gene mutation of TTR is involved in the disease, and that in geriatric amylaldosis, wild-type TTR is a protein constituting an amyloid. Since neurologic manifestations such as multiple polyneuropathy, carpal tunnel syndrome or the like are also observed as symptoms in amyloidosis, the possibility of the existence of a common mechanism with FM can be considered. In addition, since amyloid deposition also occurs secondary to an inflammatory disease such as rheumatism, the possibility that a TTR decomposition anomaly is occurring in an FM patient due to some sort of change including inflammation can also be considered.

Here, as a result of comparing and analyzing the FM patient serum samples and the healthy subject serum samples, there is a possibility that the peptides for which differences were observed in the expression amounts between the two, and the proteins and peptides described above which these peptides are derived from are related to the ethiology or pathology of the FM patients. Consequently, there is a possibility that the change in the expression amounts of these peptides can be used in the diagnosis or test for FM, by recognizing it. In addition, there is also the possibility, in a study with the purpose of exploring and developing an FM drug, that the effect of the drug on FM can be evaluated or assessed with the expression amounts of these peptide as an indicator.

### INDUSTRIAL APPLICABILITY

As described above, according to the method of the present invention, peptides which expression varies in the blood of fibromyalgia patients compared to healthy subjects were detected and for some among them, the proteins or peptides they are derived from could be identified. Consequently, the present invention is useful as a method for diagnosing or testing for fibromyalgia or for evaluating or assessing a fibromyalgia drug, by subjecting a patient's serum to peptide analysis using these identified peptides an indicator (biomarker) of fibromyalgia,

## Claims

1. A method for diagnosing or testing for fibromyalgia, comprising: measuring peptides in a blood by mass spectrometry; and using, as an indicator, a peptide having a mass-to-charge ratio (m/z) of 650,0, 656.0, 657,0, 666,0, 672.0, 679.4, 685.4, 737.5, 795.6, 810.6, 825.5, S53.7, 854.1, 861.2, 861.5, 861.5, 890.0,911.8 912.1,921.4, 942.6, 949,3, 964.6, 969.9, 970,5, 1027.9, 1028.7, 1042.5, 1055.9, 1085.9, 1086.7, 1144.8, 1741.1789.60, 1898.8, 1944.7, 2012.2, 2013.7, 2030.8, 2034.1, 2046.6, 2053.4, 2056.1, 2071.2, 2073.9, 2083.6, 2118.0, 2130.2, 2187.2, 2212.0, 2253.9, 2268.5, 2270.7, 2359.5, 2534.1, 2570.5, 2600.8, 2604.1, 2625.4, 2726.2, 2819.4, 3325.9, 3364.2 or 3380.1.

2. A method for diagnosing or testing for fibromyalgia, comprising: measuring peptides in a blood by mass spectrometry; and using, as an indicator, a peptide having a mass-to-charge ratio (m/z) of 615.6,637.4, 1020,6, 1021.3, 1061.6, 1077.8, 1206.8, 1208.0, 1262.0, 1350.7, 1350.8, 1419.6,1420.4, 1465,9, 1467,6, 1521.0, 1547.6, 1562.9, 1563.0, 1564.5, 1618.9, 1972.2, 2452.7, 2452.8, 2454.6, 2646.9, 2770.5, 2934.0, 2981.7, 3193.1 or 3263.9,

3. A method for diagnosing or testing for fibromyalgia using, as an indicator, a peptide in blood derived from high molecular weight kininogen, fibrinogen, inter-alpha-trypsin inhibitor H4, apolipoprotein E, complement C3f or transthyretin A chain.

4. A method for diagnosing or testing for fibromyalgia using, as an indicator, a peptide in blood corresponding to an amino acid sequence from position 438 to 456, position 439 to 456, position 440 to 456 or position 458 to 477 of high molecular weight kininogen.

5. A method for diagnosing or testing for fibromyalgia using, as an indicators, a peptide in blood corresponding to an amino acid sequence from position 19 to 35, portion 20 to 35, position 21 to 35, position 22 to 35, position 24 to 35, position 26 to 35 or position 28 to 35 of fibrinogen alpha chain.

6. A method for diagnosing or testing for fibromyalgia using, as an indicator, a peptide in blood corresponding to an amino acid sequence from position 626 to 642 or position 669 to 687 of inter-alpha-trypsin inhibitor H4.

7. A method for testing for fibromyalgia using, as an indicator, a peptide in blood corresponding to an amino acid sequence from position 212 to 232 of apolipoprotein E.

8. A method for diagnosing or testing for fibromyalgia using, as an indicator, a peptide in blood corresponding to an amino acid sequence from position 9 to 16 of complement C3f.

9. A method for diagnosing or testing for fibromyalgia using, as an indicator, a peptide in blood corresponding to an amino acid sequence from position 101 to 123 of transthyretin A chain.

10. A method for diagnosing or testing for fibromyalgia using, as an indicator, a peptide in blood the amino acid sequence of which is any one of SEQ ID NO. to 15.

11. A method for evaluating or assessing a fibromyalgia drug using, as an indicator, the peptide according to any one of claims 1 to 10.

12. A fibromyalgia drug evaluated or assessed by the evaluation or assessment method according to claim 11.
